# EUROPEAN PATENT APPLICATION

(11) **EP 3 257 499 A1**
(43) Date of publication of application: **20.12.2017**
(21) Application number: 16460037.1
(22) Date of filing: 17.06.2016
(51) Int. Cl.: A61K 9/16, A61K 31/506

(54) **PROCESS FOR PREPARATION OF IMATINIB METHANESULFONATE CAPSULES**

(71) Applicant: Vipharm S.A., 05-850 Ozarów Mazowiecki (PL)
(72) Inventor: Gluch, Marcin, 05-270 Marki (PL); Wierzchucka, Katarzyna, 03-149 Warszawa (PL); Nowak, Mariusz, 03-986 Warszawa (PL); Zaleska, Grazyna, 05-120 Legionowo (PL); Fraczek, Urszula, 05-261 Marki (PL); Markiewicz, Katarzyna, 02-495 Warszawa (PL); Soltysiak, Krzysztof, 04-680 Warszawa (PL); Myszkorowski, Jan, 02-904 Warszawa (PL); Bartosiewicz, Dariusz, 03-179 Warszawa (PL); Patek, Jerzy, 03-992 Warszawa (PL)
(74) Representative: Krzywdzinska, Ewa

(57) **Abstract**

The process for preparation of hard gelatin capsules of imatinib methanesulfonate having a decreased volume consists in that the imatinib methanesulfonate granulate designated for the filling the capsules is deprived of a fraction of the particles size below 0.250 mm, extragranular ingredients are added and thus obtained blend is used to fill the hard gelatin capsules. The object of the invention is also the medicinal product in the form of hard gelatin capsules comprising imatinib methanesulfonate in an amount corresponding to the 100 mg imatinib dose in capsule size number 4 and the 400 mg imatinib dose in capsule size number 0el. The capsules are characterized by more advantageous dissolution profiles and better bioavailability in comparison with capsules manufactured from non-separated granulate.

## Description

### Field of the invention

The present invention relates to a process for preparation of imatinib methanesulfonate capsules having the volume decreased when compared to the known products and to the product in the form of hard gelatin capsules manufactured by this method.

### Background of the invention

4-(4-Methylpiperazin-1-ylmethyl]-N-[4-methyl-3-[4-(pyridin-3-yl)pyrimidin-2-ylamino]-phenyl]benzamide, known under an international name of (INN) imatinib, is a selective tyrosine kinase inhibitor. The compound is disclosed in EP 0564409 B1 and the corresponding US 5,521,184 B2 patents. Imatinib is widely used in the treatment of cancer, especially in the treatment of chronic myeloid leukemia and acute lymphoblastic leukaemia, both in adults and children and in the treatment of gastrointestinal tumors in adults. The medicinal product, approved to be used for humans, available among others under the trade name of Glivec® (manufactured by Novartis Europharm Ltd.), comprises imatinib methanesulfonate. Imatinib methanesulfonate and its pharmacologically equivalent α and β crystalline polymorphic forms are described in EP 0998473 B1 patent specification.

In accordance with the Summary of Product Characteristics issued on 18 August 2009 by the European Medicines Agency (Summary of Product Characteristics, 18/08/2009; http://www.ema.europa.eu/ema)? imatinib is administered orally in very high doses. The recommended daily dose in the maintenance therapy of chronic myeloid leukaemia in adults is 400 mg, and in the accelerated phase and in blast crisis of this disease - 600 mg. The dose of 400 mg/day may be increased even up to 800 mg/day. The doses administered in the leukaemia treatment with children are not smaller. In the chronic phase, the recommended dosage is 340 mg/m² of the body surface/day. The doses even up to 800 mg/day may be administered.

The medicinal product Glivec® has been approved in the form of hard gelatin capsules of 50 mg and 100 mg and film-coated tablets comprising 100 mg and 400 mg of imatinib methanosulfonate calculated into a free base.

Imatinib methanesulfonate active substance, especially in α-crystal form, is characterized by very unfavorable rheological properties from the point of view of manufacturing technology of solid oral dosage forms. As it is disclosed in patent description EP 0998473 B1, the α-Form crystals are needle-shaped, hygroscopic, they have electrostatic properties and very poor flow characteristics.

Due to the necessity of administration of high doses of the medicine in treating patients, in connection with generally known unfavorable rheological properties of an active substance, unit dosage forms of imatinib methanesulfonate have very large sizes.

An example of EP 0998473 B1 provides for a description of the composition, which, even though it comprised imatinib methanesulfonate in a more compact β-crystal form, required a capsule of size number 1 (bulk volume: 0.50 mL, total length: 19.4 mm, maximum diameter: 6.91 mm) to be used to fill in the dose of 100 mg of imatinib.

Similarly, in the case of using a composition described in the International Patent Application WO 01/47507, for the dose of 100 mg of imatinib, capsule of size number 1 was necessary to use.

The European Medicines Agency (EMA) authorized several generic medicinal products containing imatinib methanesulfonate to be launched into the market in the European Communities, including Imatinib Actavis 50 mg hard gelatin capsules of size number 3, 100 mg hard gelatin capsules of size number 1 and 400 mg hard gelatin capsules of size number 00, and Imatinib medac 100 mg hard gelatin capsules of size number 3 and 400 mg hard gelatin capsules of size number 00, as well as Imatinib Teva 100 mg hard gelatin capsules of size number 1 and hard gelatin capsules of size number 00. [http://www.ema.europa.eu/ema].

In the Guidelines for Industry ["Size, Shape, and Other Physical Attributes of Generic Tablets and Capsules"; Guidance for Industry, June 2015], the Food and Drug Administration (FDA) states that "the size and shape of tablets and capsules affect the transit of the product through the pharynx and esophagus and may directly affect patient's ability to swallow drug products". "Larger tablets and capsules have been shown to have a prolonged esophageal transit time", which can lead to a disintegration of the tablet or capsule in the esophagus and cause injury to the esophagus, resulting in pain and esophagitis and the potential for serious sequelae.

Patients, especially children and elderly, have serious difficulties in swallowing large capsules and tablets, the more so, the therapy often requires taking few such unit forms daily. The anticancer imatinib therapy itself causes the patients' nausea, strong vomiting reflexes and vomiting. The swallowing of very large tablets or capsules becomes an additional burden. It is true that the informative material relating to the medicinal product includes the instruction according to which "for patients, unable to swallow the tablet or the capsule, their content may be diluted in a glass of either water or apple juice", but the fact that the active substance is exceptionally bitter is not taken into account. The patient, especially a child, is not able to take very bitter liquid every day.

That is why the pharmaceutical manufacturers search for solutions which would eliminate or just mitigate nuisances related to a necessity to swallow imatinib tablets or capsules of a very large size, and by the same, would ensure the patients' compliance.

The attempts go towards several directions: In one approach, the efforts are made to develop a dosage forms that would mask a bitter taste of imatinib which allows to suspend a tablet or sachet content in water and then to drink the solution or dispersion. In the patent specifications KR 2014/0065862 A and CN 101401797 A, the quick-dissolving effervescent preparations were disclosed, in which a bitter taste of imatinib is attempted to reduce by the addition of sweeteners and flavoring agents.

In the other approach, the attempts aimed at decreasing the volume of oral dosage form by limiting the amount of introduced inactive excipients. To this extent, a typical example is a "high drug load" tablet described in the patent publication EP 1501485 B1. This tablet comprises approximately 52% of an active substance. However, a β-crystal form of imatinib methanesulfonate of much more advantageous flow properties than the α-crystal form was used to manufacture this tablet.

In the publication of patent application PL 394169 A1 a solid blend to fill in the unit dosage forms was described. This blend is obtained by dry granulation of imatinib methanesulfonate, optionally with admixture of pharmaceutical excipients, preferably by compacting or slugging and blending the resulting granulate with a lubricant and optionally with glidant, if any. It was stated that the prepared blend can be used for filling capsules of size number 3 comprising 100 mg dose and capsules of size number 00 comprising the dose of 400 mg of imatinib.

In yet another approach, the efforts have been made to improve the flow properties of the granulate used to manufacture the final pharmaceutical unit dosage forms. Such approach is presented in the published Polish patent application PL 394559 A1. The inventors of PL 394559 A1 managed to pack the dose of 400 mg of imatinib (as methanesulfonate) into hard gelatin capsules of sizes numbers 0 or 0el. This objective was achieved by granulating the active substance, using absolute ethyl alcohol or a solution of polyvinylpyrrolidone in absolute ethyl alcohol or a suspension of colloidal silica in absolute ethyl alcohol. The obtained granulate, when sieved through 1,0 mm sieve, is characterized by such grain size distribution in which a fraction of a maximum share has particle size within the range from 50 µm to 400 µm, and its bulk density is not less than 0,6 g/mL. In the enclosed examples there are described granulates in which the fraction of a maximum share was within the range from 70 to 300 µm or from 100 to 400 µm.

The process described in the patent application PL 394559 A1 is burdened with great inconvenience, namely, absolute ethyl alcohol is used for granulation. It creates a danger of fire. In the patent specification there are no data relating to the parameters of obtained capsules. It is not known whether they meet pharmacopoeial requirements like dose uniformity, dissolution rate and other requirements, or whether they meet the bioavailability criteria.

The International patent publication WO 2011/160798 A1 provides for the wet granulation process of imatinib methanesulfonate using water only. After drying, the granulate was sieved through 20 mesh (0.84 mm) sieve; and, after adding extragranular ingredients, it was sieved through 30 mesh (0.6 mm) sieve. The obtained granulate was used solely to manufacture the tablets.

According to the International patent publication WO 2012/019633, the granulate of imatinib methanesulfonate was obtained in the process of wet granulation using either alcohol (ethanol and isopropyl alcohol) or water. Granulate after drying was first sieved through 3.0 mm sieve, and then through the 1.0 mm sieve. The obtained dry granulate had an average size of particles from 250 µm to 800 µm. The particle size distribution in the total mass of granulate has not been disclosed, neither any data relating to the properties of the manufactured granulate were given. There is no information concerning its suitability to manufacture capsules.

In the foregoing two publications, only water was used as a wetting agent in the granulation process. Indeed, it allows to obtain the granulate of very good handling properties; however, the process itself proves technologically very burdensome due to significant viscosity of the particles, their clumping and depositing sticky mass on the walls of equipment using even a minimum amount of water (like in example 1 of the PL 394981 A1), which causes significant losses of active substance and a need of continuous cleaning of apparatuses.

The International patent publication WO 2012/080703 discloses the process of filling the capsules with the granulate obtained by spraying the blend of imatinib methanesulfonate and crospovidone with a mixture of isopropyl alcohol and water, sieving the dried granulate through the sieve and adding crospovidone and magnesium stearate. The 400 mg doses of imatinib methanesulfonate calculated into free base were filled in capsules of size number 00, and the 100 mg doses were filled in capsules of size number 3. The rheological properties of the granulate nor the quality data of the manufactured imatinib capsules were not disclosed.

In the International patent publication WO 2012/087257 are disclosed blends to fill the capsules. These blends were prepared by blending of imatinib methanesulfonate in α-crystalline form with inactive ingredients. In these blends, the particle size distribution of the active substance was characterized by the ratio D(90)/D(10) within the range from 5 to 20. The use of imatinib satisfying this criterion should ensure a quick release of the active substance from capsules. In the formulations where the ratio D(90) to D(10) exceeded a specified range of 5-20, a dissolution rate was significantly worse. However, the analysis of the effect of an absolute size of active substance particles (Tables 1.2, 1.3, 2.2 and 2.3) on the dissolution rate does not show any correlation between the contents of a fraction of the smallest size of particles and the release rate. Also, it is not possible to presume the relation of the particle size to the dissolution rate within the range D(90). The patent description does not provide for any information about the method of obtaining the active substance of the ratio D(90)/D(10) within the range of 5-20.

In the patent application PL 394981 A1 there is described the method of manufacturing of granulate of imatinib methanesulfonate in an α-crystal form The obtained granulate contains an active substance as the only ingredient, i.e. it is the granulate without any inactive excipients. The granulate of advantageous flowability was obtained as a result of using the mixture of isopropyl alcohol and water for granulating the active substance. The most optimal flow properties of the granulate were searched by selecting an appropriate proportion of isopropyl alcohol to water in the solution used for granulating.

In the quoted patent description, it was stated that a granulate grain size is not a critical parameter and that it may amount from about 0,5 mm to about 3 mm. The grain size compliant with a designation of the granulate was achieved by using sieves with the openings' diameter usually applied during granulation process. 1 mm sieves were used to obtain the granulate used for the manufacture of tablets or capsules; whereas the granulate used to manufacture sachet dosage forms could have larger grain size.

The granulate obtained by the method described in PL 394981 A1 was used to fill hard gelatin capsules. Even with the addition of very small amounts of excipients (e.g. colloidal silica and magnesium stearate in a total amount less than 1.3% w/w of the total mass), the blend containing 100 mg or 400 mg imatinib calculated into free base had to be filled in capsule numbers 3 and 00, respectively.

Hard capsules are a convenient and preferably manufactured solid oral dosage form of medicines. They have several advantages. They are characterized by high stability and mechanical strength, they protect well active substance, they provide a possibility to use less excipients than are needed for production of tablets, they effectively mask an unpleasant taste or smell. Moreover, they have elongated and oval shape, and thus are easier to swallow.

On the other side, it is a common knowledge that a critical parameter in the capsule filling process includes rheological properties, including in particular the flowability of the blend used for filing process. In the case of imatinib methanesulfonate, specifically in its α-crystal form, its free flowing and electrostatic properties cause greatest difficulties.

Now, it has surprisingly appeared that it is possible to significantly improve rheological properties of the imatinib methanesulfonate granulate not only by selecting optimum composition of the solution used to moisten a dry active substance during the granulation process, but also by eliminating smallest particles from the granulate. The granulate obtained in accordance with the application PL 394981 A1 was deprived of a dusty fraction by sieving it through sieves with openings over 0.1 mm. The improved granulate, after mixing it with small amount of excipients, is suitable to manufacture imatinib capsules of smaller dimensions. This mitigates a nuisance of swallowing capsules with patients, and gives a relief specifically for pediatric patients.

In addition, it has surprisingly appeared that an intended purpose of packing a required dose of imatinib methanesulfonate into smaller capsules can be achieved starting from the initial (not separated) granulate of a lower bulk density and a worse flowability. It was experimentally established that for instance the 100 mg imatinib methanesulfonate dose may be filled in the number 4 capsule by eliminating a dusty fraction from the granulate of a bulk density not exceeding 0.40 g/mL.

It has also unexpectedly turned out that it is possible to improve quality of imatinib capsules manufactured using the granulate obtained by the process of the invention. The capsules manufactured by the process of the invention have smaller dimensions and also are characterized by a better profile of a dissolution rate in media of various pH values, and, most of all, better bioavailability, as confirmed by bioequivalence trials conducted in comparison to a reference product.

Until now, the method for improving flow properties of imatinib methanesulfonate granulate by removing the fraction of smallest particle size has not been described.

### Summary of the Invention

This present invention provides for a process for preparation of imatinib methanesulfonate hard capsules having a reduced volume, wherein:
a) imatinib methanesulfonate granulate designated to fill the capsules is deprived of a fraction of a particle size below 0.250 mm;
b) the granulate obtained in step a) is blended with extragranular excipients;
c) the blend from step b) is filled into hard gelatin capsules.
In the preferred embodiment, the imatinib methanesulfonate granulate is deprived of the smallest size particles by separating it on the sieves with 0.1 mm to 0.250 mm openings.

In one embodiment, the imatinib methanesulfonate granulate is separated on the sieve with 0.250 mm openings.

In another embodiment, the imatinib methanesulfonate granulate is separated on a sieve with 0.125 mm openings.

In still another embodiment, the granulate of imatinib methanesulfonate in α-crystal form is used for the separating process.

In the preferred embodiment of the invention, for the separating process is used the granulate of imatinib methanesulfonate in α-crystal form, manufactured by granulating imatinib methanesulfonate in α-crystal form by using a mixture of isopropyl alcohol and water.

In the more preferred embodiment of the invention, for the separating process is used the granulate of imatinib methanesulfonate in α-crystal form, manufactured by granulating imatinib methanesulfonate in α-crystal form by using a mixture of isopropyl alcohol and water in the proportion of from 80:20 to 20:80 w/w.

In the preferred embodiment of the invention, the used extragranular excipients include substances which improve flow properties of the granulate, like glidants and antielectrostatic agents, selected from the group including, among others, stearic acid, stearic acid salts with alkaline earth metals, like calcium stearate or magnesium stearate, colloidal silica, talc and other known additives.

In the preferred embodiment, a blend of imatinib methanesulfonate from step b) in an amount corresponding to the 100 mg imatinib dose is filled into a capsule number 4.

In another preferred embodiment, a blend of imatinib methanesulfonate from step b) in an amount corresponding to the 400 mg imatinib dose is filled into a capsule number 0el.

Another aspect of the invention is the product manufactured in accordance with the invention in the form of hard gelatin capsules which comprise imatinib methanesulfonate in an amount corresponding to the 100 mg imatinib dose in a capsule size number 4.

Another aspect of the invention is the product manufactured in accordance with the invention in the form of hard gelatin capsules which comprise imatinib methanesulfonate in an amount corresponding to the 400 mg imatinib dose in a capsule size number 0el.

### Description of the Figures

Fig. 1 represents the result of sieve analysis of imatinib methanesulfonate granulate from a Reference Example 1, separated on the sieve with 0.125 mm openings.
Fig. 2 represents the result of sieve analysis of imatinib methanesulfonate granulate from a Reference Example 1, separated on the sieve with 0.250 mm openings.

### Detailed description of the invention

In the present description, the imatinib methanesulfonate capsule of a reduced volume is defined as a capsule which contains the same amount of active substance as those known in the state of art, but placed in the capsule of a smaller size.

The capsule size in the context of this description is understood as the size corresponding to numbering system adopted in the international turnover and also accepted by the medicine registration agencies. It relates to the bulk volume which may be comprised in a capsule of a specific size number.

The bulk volumes for each capsule size are specified in the following table:

| Capsule size number | Bulk volume (mL) of hard gelatin capsules |
|---|---|
| 000 | 1.37 |
| 00el | 1.02 |
| 00 | 0.91 |
| 0el | 0.78 |
| 0 | 0.68 |
| 1el | 0.54 |
| 1 | 0.50 |
| 2el | 0.41 |
| 2 | 0.37 |
| 3 | 0.30 |
| 4 | 0.21 |
| 5 | 0.13 |

In conventional preparations authorized for marketing, the smallest hard gelatin capsules comprising 100 mg imatinib methanesulfonate active substance calculated into free base are of size number 3, and the 400 mg capsules are of size number 00.

In the solution according to the invention, the same doses of 100 mg and 400 mg imatinib (as methanesulfonate) are filled into the capsules of size numbers 4 and 0el respectively.

The imatinib methanesulfonate granulate means in the present description, the granulate comprising imatinib methanesulfonate as the only compound, without any inactive excipients. Preferably, it is the imatinib methanesulfonate granulate in α-crystal form, manufactured by the method described in the patent application PL 394981 A1.

In the present invention, the capsule means hard, two-parts capsules, cylindrical or oval, made of gelatin or hydroxypropylmethyl cellulose.

During pre-formulation experiments on a possibility to fill a required dose of imatinib methanesulfonate into capsules smaller than currently available capsules, the granulates were manufactured using the process described in PL 394981 A1, and the attempts were focused on the possibility to obtain as big as possible bulk density, searching for an optimal composition of a solution used for granulating process and changing parameters of mixing a wet mass. However, it was not possible to fill a 100 mg dose into capsules of the size smaller than number 3, and the 400 mg dose into capsules of the size smaller than number 00. The use of isopropyl alcohol/water solutions containing high contents of water for the granulation process caused technical difficulties as the viscosity of the wet mass was too high.

It has unexpectedly appeared, that the object to fill the required dose of imatininb methanesulfonate into the capsule of smaller volume could be achieved by eliminating the particles of the smallest size, i.e. dusty fraction from the granulate.

It has turned out that if a dusty fraction is removed from the granulate manufactured in accordance with the process described in PL 394981 A1, its rheological properties improve significantly and the 100 mg imatinib methanesulfonate capsules of size number 4 and 400 mg imatinib methanosulfonate capsules of size number 0el may be easily obtained, thereby patients feel an improved comfort when they take the medicine.

The dusty fraction may be removed from the granulate by any method known in the industry, as for instance by manual sieving or using mechanical sieving equipment, for instance apparatuses with vibratory action or by sieving with the use of a fanner. The selection of the machine depends on the process scale. Laboratory shakers may be used, as well as industrial equipment, such as machine with vibration screen recommended for separating of dusts.

In an embodiment of the invention, the fraction of the smallest size of particles is removed by sieving the granulate on sieves. The granulate is sieved using a sieve with size openings above 0.1 mm preferably using the sieve with 0.125 mm openings, and even more preferably using the sieve with 0.250 mm openings.

During the separating process, a grain size distribution in granulates changes and the change depends on size openings in the sieve used for separating from dusty fraction.

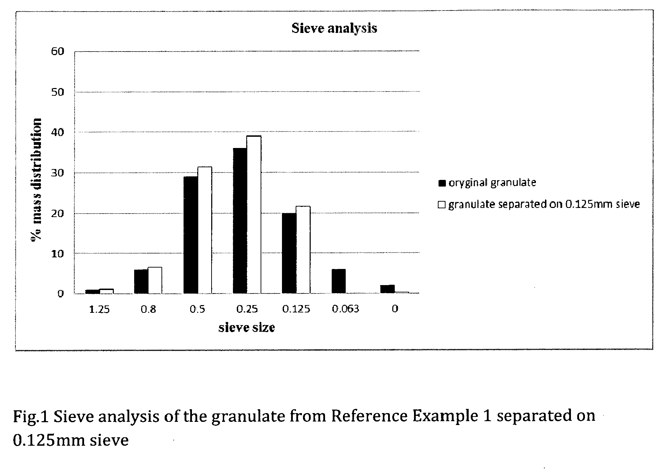

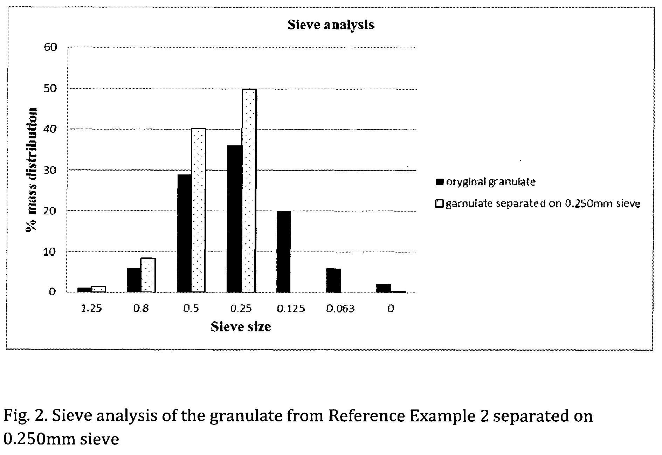

An example grain size distribution in the granulates sieved through 0.125 mm and 0.250 mm sieves is depicted in Table 1 and in Fig. 1 and Fig. 2.

**Table 1. The grain size distribution in the granulates separated on sieves with of 0.125 mm and 0.250 mm openings.**

| | Fraction trapped above the screen (% by weight) | | | | | | |
|---|---|---|---|---|---|---|---|
| | 1.25 mm | 0.8 mm | 0.5 mm | 0.25 mm | 0.125 mm | 0.063 mm | Collector (sscreen 0) |
| Initial granulate | 1 | 6 | 29.0 | 36.0 | 20 | 6 | 2 |
| Granulate separated on 0.125 mm sieve | 1.1 | 6.5 | 31.5 | 39.0 | 21.7 | 0.0 | 0.2 |
| Granulate separated on 0.250 mm sieve | 1.4 | 8.3 | 40.2 | 49.9 | 0.0 | 0.0 | 0.1 |

The plots on the figures clearly indicate that the granulates obtained by the process of the invention do not include the fraction of the smallest particles and that the grain size distribution depends upon the size of the openings in the sieve used for separating.

The granulates manufactured using the method in accordance with PL 394981 A1 and then improved during separating process have much more advantageous rheological properties. Their bulk density and flowability improve. Final values of bulk density and flowability depend upon properties of the original granulate and a type of sieve applied for separating from dusty fraction. As depicted in Table 2, the best results are obtained by sieving through 0.250 mm sieve. In the practical embodiment it has appeared that the goal of the invention may be obtained already using the granulate separated on 0.125 mm sieve.

The separated dusty fraction is returned to the manufacturing process of the initial granulate, and, therefore, the sieving process does not involve any losses of the active substance.

**Table 2. Bulk density change of granulates separated on 0.125 mm and 0.250 mm sieves**

| Bulk density (g/mL) | | |
|---|---|---|
| Granulate before separating | Granulate separated on 0.125 mm sieve | Granulate separated on 0.250 mm sieve |
| 0.38 | 0.44 | 0.48 |
| 0.48 | 0.50 | 0.53 |

Typical extragranular excipients which improve flowability of granulates, like glidants, lubricants and antielectrostatic agents, like stearic acid or its salts with alkaline earth metals, e.g. calcium stearate or magnesium stearate, colloidal silica, talc and other known additives, which play a similar role, may be added to the granulates designated to fill in the capsules.

Thus, the capsule manufactured by the process of the invention contains, apart from imatinib methanesulfonate active substance, only ingredients necessary to obtain the blend flowability and to avoid granulate adhering to the walls of manufacturing equipment during mixing and filling in empty capsules.

For the purposes of comparison, Glivec^{®} 50 mg capsule contains microcrystalline cellulose as bulking agent, crospovidone as disintegrant and anhydrous colloidal silica and magnesium stearate as glidants and lubricants.

In the embodiment of the intention, as small as possible amounts of excipients are used to achieve the smallest possible total bulk mass of the capsule.

Exemplary capsules disclosed in the patent application PL 394981 A1 contain only colloidal silica and magnesium stearate in an amount of 1.26% w/w of the total mass of the blend used to fill in the capsules.

In the embodiment of the invention, as a result of improving the imatinib methanesulfonate granulate by removing the dusty fraction, it was possible to decrease even more the contents of excipients. Only glidants, like stearic acid or stearates of alkali metals are added in amounts below 1% w/w. In preferred embodiments only magnesium stearate is added to the granulate.

An unexpected additional advantage of the method of the invention is that the capsules of clearly better quality are obtained. The capsules manufactured from the granulate deprived of dusty fraction not only fulfill pharmacopoeial requirements, such as contents uniformity or stability, but they are also characterized by more advantageous dissolution profiles of active substance and also better bioavailability when compared to capsules manufactured from non-separated granulate.

The invention is illustrated by the following examples which do not limit its scope.

### Examples

### Analytic methods

Rheological properties of granulates were assayed in Erweka Granulate & Powder Flow Tester, type GTB with the measurement of the angle of repose.

The bulk density was assayed in accordance with Ph.Eur., Chapter 2.9.34 (method 1). 50 g of granulate is introduced without compacting into the 250 mL graduated cylinder and the volume of the tested granulate is read.

The flowability (flow time) was assayed in accordance with Ph.Eur. Chapter 2.9.16 in Erweka Granulate & Powder Flow Tester, type GTB. The 100 g granulate is introduced without compaction into a dry funnel, whose bottom opening has been previously blocked. The bottom opening of the funnel is unblocked and the time needed for the sample to flow out of the funnel is measured.

Flowability is defined as the time needed to flow of 100 g of granulate through the opening of 15 mm diameter.

Particle size distribution in the granulate was estimated by analytical sieving in the analyser of LPzE-2e type (Multiserv Morek), consisting of a column with the set of sieves placed in the laboratory sieve shaker. The amount of 100 g granulate was subjected to fractionation on sieves of openings diameters of 1.25 mm, 0.80 mm, 0.50 mm, 0.25 mm, 0.125 mm and 0.063 mm. The following shaking parameters were used: time - 12 minutes; vibration frequency - 50 Hz; vibration amplitude - 43%. Each sieve was weighted and the mass retained on each sieve was determined.

Dissolution rate of active substance from capsules was determined in accordance with Ph. Eur., Chapter 2.9.3, Apparatus 2. Tests were carried out in paddle apparatus, with rotation speed of 50 rpm, concurrently for 6 capsules. The 0.01% hydrochloric acid solution (pH 2), acetate buffer of pH 4,5 and phosphate buffer of pH 6,8 were applied as dissolution media.

### Reference Example 1. Manufacturing of imatinib methanesulfonate granulate

Imatinib methanesulfonate in its α-crystal form (524 g) was charged into highspeed granulator Glatt TMG 1/6 with 2 dm³ working vessel. The content of the vessel was mixed for one minute by a mixer with the rotor speed 200 rpm. Then the process of spraying the mass with the isopropyl alcohol - water mixture (in the proportion 70:30 of weight parts) was started. The solution was added with the speed of 20 g/min by peristaltic pump. Feeding with the solution was continued for 3 minutes, with the main mixer speed switched on 200 .. In total 100 g of solution was added. Upon the completion of adding of the solution the chopper working with the speed of 1500 rpm was started. The process of final granulation was carried out for 1 minute. The received granulate was loaded onto tray drying oven (Memmert GmbH) and was dried up to moisture contents below 0.5% w/w. The dried granulate was calibrated initially on the 1.6 mm sieve using Erweka oscillating granulator, and then the granulate was calibrated on the 1.0 mm sieve. Obtained granulate had bulk density of 0.38 g/ml and flowability of 7.2 sec.

### Reference Example 2. Manufacturing of imatinib methanesulfonate granulate

The imatinib methanesulfonate granulate was manufactured by the process described in the reference example 1, but the mixture of isopropyl alcohol - water in the proportion of 50:50 of weight part) was applied for moistening. The obtained granulate was characterized by bulk density of 0.48 g/ml and flowability of 6.2 sec.

### Example 1. The method of manufacturing imatinib methanesulfonate granulate deprived of dusty fraction.

The granulate manufactured by the process described in Reference Example 1 was separated from dusty fraction in a laboratory siever LPzE-2e (Multiserv Morek). The siever was set on the following working programme:
Cycle time: 12.00 minutes
Vibration interval: 0.01 minute
Vibration frequency: 50 Hz
Vibration amplitude: 43%.
The 0.125 mm or 0.250 mm sieves were applied.

Flow properties of granulates manufactured in Example 1 are presented in Table 3.

**Table 3. Flow properties of granulates manufactured in Example 1**

| Parameter | Granulate of Reference Example 1 (without separating) | Granulate of Example 1 (separated on 0.125 mm sieve) | Granulate of Example 1 (separated on 0.250 mm sieve |
|---|---|---|---|
| Bulk density (g/mL) | 0.38 | 0.44 | 0.48 |
| Flowability (sec) | 7.2 | 6.8 | 6.5 |

### Example 2. The method of manufacturing imatinib methanesulfonate granulate deprived of dusty fraction.

The granulate manufactured by the process described in the Reference Example 2 was separated from dusty fraction on 0.125 mm or 0.250 mm sieve. The procedure described in Example 1 was applied.

Flow properties of granulates manufactured in Example 2 are presented in Table 4.

**Table 4. Flow properties of granulates manufactured in Example 2.**

| Parameter | Granulate of Reference Example 1 (without separating) | Granulate of Example 1 (separated on 0.125 mm sieve) | Granulate of Example 1 (separated on 0.250 mm sieve) |
|---|---|---|---|
| Bulk density (g/ml) | 0.48 | 0.50 | 0.53 |
| Flowability (sec) | 6.2 | 5.8 | 5.6 |

### Reference Example 3. Manufacturing of hard gelatin capsules from non-separated granulate.

The capsules of imatinib methanesulfonate in the doses of 100 mg and 400 mg were obtained from the granulate manufactured in accordance with Reference Example 1. The capsules had the following composition:

| | 100 mg dose [mg] | 400 mg dose [mg] |
|---|---|---|
| Imatinib methanesulfonate granulate from reference example 1 | 119.47 | 477.88 |
| Colloidal silicon dioxide | 0.75 | 3.00 |
| Magnesium stearate | 0.78 | 3.12 |
| Total mass | 121.00 | 484.00 |

The imatinib methanesulfonate granulate obtained in Reference Example 1 was blended in container blender made of stainless steel, with the following substances previously sieved through 0.5 mm sieve: colloidal silicon dioxide and magnesium stearate. The rotation speed: 4 rpm, mixing time: 10 minutes. The obtained blend was used to fill in CAPSUGEL^{®} hard gelatin capsules of size numbers "3" and "00", for the 100 mg and 400 mg doses respectively. The encapsulation process was carried out in InCap semi-automatic laboratory encapsulation machine, manufactured by Bonapace. During the capsule filling process, the capsules filling mass was controlled.

### Example 3. Manufacturing of 100 mg hard gelatin capsules from granulate deprived of dusty fraction.

**Composition of 100 mg capsules:**

| | |
|---|---|
| Imatinib methanesulfonate from Example 1 separated on 0.125 mm sieve | 119.47 mg |
| Magnesium stearate | 0.53 mg |
| Total | 120.00 mg |

Magnesium stearate was sieved through the 0.5 mm sieve and was blended with imatinib methanesulfonate granulate manufactured in Example 1 and separated on 0.125 mm sieve. The blending was carried out in a container blender made of stainless steel for 10 minutes with the rotation speed of 4 rpm. The obtained blend was used to fill CAPSUGEL^{®} hard gelatin capsules size number 4. The encapsulation process was carried out in Bonapace InCap semi-automatic laboratory encapsulation machine. During the encapsulation process, the capsule filling mass was controlled.

The manufactured capsules are characterized by dissolution rate assayed with a pharmacopoeial method specified in Table 5.

**Table 5. The dissolution rate of active substance from 100 mg capsules of imatinib metanesulfonate manufactured by the process described in Example 3 to three pharmacopoeial media, in comparison with the dissolution rate for the capsules manufactured using non-separated granulate.**

| **Medium: 0,01 M HCl (pH 2,0)** | **after 5 min** | **after 10 min** | **after 15 min** | **after 30 min** |
|---|---|---|---|---|
| Capsules from Reference Example 3 (without granulate separating) | 57.4% | 99.0% | 102.4% | 103.1% |
| Capsules from Example 4 (granulate separated on 0.125 mm sieve) | 57.6% | 98.1% | 103.8% | 104.1% |

| **Medium: acetate buffer (pH 4,5)** | **after 5 min** | **after 10 min** | **after 15 min** | **after 30 min** |
|---|---|---|---|---|
| Capsules from Reference Example 3 (without granulate separating) | 36.5% | 83.7% | 93.5% | 95.5% |
| Capsules from Example 3 (granulate separated on 0.125 mm sieve) | 41.2% | 86.4% | 101.1% | 102.9% |

| **Medium: phosphate buffer (pH 6,8)** | **after 5 min** | **after 10 min** | **after 15 min** | **after 30 min** |
|---|---|---|---|---|
| Capsules from Reference Example 3 (without granulate separating) | 36.5% | 83.7% | 93.5% | 95.4% |
| Capsules from Example 3 (granulate separated on 0.125 mm sieve) | 41.2% | 88.2% | 100.1% | 101.8% |

### Example 4. Manufacturing of 400 mg hard gelatin capsules from granulate deprived of dusty fraction.

**Composition of 400 mg capsules:**

| | |
|---|---|
| Imatinib methanesulfonate from Example 2 separated on 0.250 mm sieve | 477.88 mg |
| Magnesium stearate | 2.12 mg |
| Total | 480.00 mg |

The imatinib methanesulfonate granulate from Example 2 separated on 0.250 mm sieve was blended with magnesium stearate; the blend was used to fill the CAPSUGEL^{®} hard gelatin capsules of size number 0el. The process was carried out by the method described in Example 3.

The capsules manufactured by the method in accordance with the invention are characterized by the dissolution rate in pharmacopoeial media presented in Table 6.

**Table 6. Comparative dissolution profile of imatinib 400 mg hard capsules manufactured in Example 4 vs Glivec 400 mg coated tablets in three media.**

| **Medium: 0.01 M HCl (pH 2.0)** | **after 5 min** | **after 10 min** | **after 15 min** | **after 30 min** |
|---|---|---|---|---|
| Glivec® 400 mg film-coated tablets | 44.6% | 84.0% | 97.7% | 99.8% |
| Capsules from Reference Example 3 (without granulate separating) | 34.2% | 81.7% | 97.3% | 99.6% |
| Capsules from Example 4 (granulate separated on 0.250 mm sieve) | 55.2% | 95.2% | 102.8% | 103.7% |

| **Medium: acetate buffer (pH 4.5)** | **after 5 min** | **after 10 min** | **after 15 min** | **after 30 min** |
|---|---|---|---|---|
| Glivec® 400 mg film-coated tablets | 43.2% | 81.8% | 99.4% | 104.5% |
| Capsules from Reference Example 3 (without granulate separating) | 29.0% | 71.8% | 93.9% | 99.3% |
| Capsules from Example 4 (granulate separated on 0.250 mm sieve) | 37.3% | 76.62% | 99.4% | 104.5% |

| **Medium: phosphate buffer (pH 6.8)** | **after 5 min** | **after 10 min** | **after 15 min** | **after 30 min** |
|---|---|---|---|---|
| Glivec® 400 mg film-coated tablets | 51.8% | 87.1% | 95.3% | 97.1% |
| Capsules from Reference Example 3 (without granulate separating) | 19.3% | 55.1% | 84.1% | 96.0% |
| Capsules from Example 4 (granulate separated on 0.250 mm sieve) | 36.3% | 87.9% | 100.7% | 103.3% |

*In vivo* trials: The capsules manufactured by the method of the invention are characterized also by excellent pharmacokinetic parameters. In bioavailability study performed in an open, randomized, two-sequence, two-period crossover design the capsules manufactured by the method of the invention proved to be fully bioequivalent with Glivec® medicinal product.

### Discussing the results.

Due to removing of the dusty fraction from the imatinib methanesulfonate granulate the 100 mg imatinib capsules of size number 4 and 400 mg capsules of size number 0el are provided.

The results of *in vitro* tests and *in vivo* trials presented above confirm that the capsules manufactured by the method in accordance with the invention are valuable medicinal product, fully bioequivalent with Glivec^{®} reference product manufactured by Novartis Europharm Ltd.

## Claims

1. A process for preparation of imatinib methanesulfonate hard capsules having a reduced volume, wherein :
a) imatinib methanesulfonate granulate designated to fill the capsules is deprived of a fraction of a particle size below 0.250 mm;
b) the granulate obtained in step a) is blended with extragranular excipients;
c) the blend from step b) is filled into hard gelatin capsules.

2. The process according to claim 1, wherein the imatinib methanesulfonate granulate is deprived of the smallest size particles by separating it on the sieves with 0.1 mm to 0.250 mm openings.

3. The process according to claim 2, wherein the imatinib methanesulfonate granulate is separated on the sieve with 250 mm openings.

4. The process according to claim 2, wherein the imatinib methanesulfonate granulate is separated on the sieve with 0.125 mm openings.

5. The process according to any of claims 1 to 3, wherein the granulate of imatinib methanesulfonate in α-crystal form is used for separating process.

6. The process according to claim 5, wherein the granulate obtained by granulating imatinib methanesulfonate in α-crystal form by using a mixture of isopropyl alcohol and water is used for separating process.

7. The process according to claim 6, wherein the granulate obtained by granulating imatinib methanesulfonate in α-crystal form by using a mixture of isopropyl alcohol and water in the weight ratio between 80:20 and 20:80 w/w is used for the separating process.

8. The process according to any of claims 1 to 7, wherein the used extragranular excipients comprise substances which improve flow properties of the granulate, like glidants and antielectrostatic agents.

9. The process according to claim 8, wherein excipients selected from the group comprising stearic acid, stearic acid salts with alkaline earth metals, colloidal silica, talc and other known additives are used as extragranular excipients.

10. The process according to claim 1, wherein the separated dusty fraction is returned to the process of preparation of the initial granulate.

11. The process according to any of claims 1 to 9, wherein the blend of imatinib methanesulfonate from step b) in an amount corresponding to the 100 mg imatinib dose is filled into a capsule number 4.

12. The process according to any of claims 1 to 9, wherein a blend of imatinib methanesulfonate from step b) in an amount corresponding to the 400 mg imatinib dose is filled into a capsule number 0el.

13. Medicinal product manufactured in accordance to any of claims 1 to 9, in the form of hard gelatin capsules which comprise imatinib methanesulfonate in an amount corresponding to the 100 mg imatinib dose in a capsule size number 4.

14. Medicinal product manufactured in accordance to any of claims 1 to 9 in the form of hard gelatin capsules which comprise imatinib methanesulfonate in an amount corresponding to the 400 mg imatinib dose in a capsule size number 0el.
